(19) Europäisches Patentamt
European Patent Office
Office européen des brevets

(11) Publication number: 0 240 122 B1

# (12) EUROPEAN PATENT SPECIFICATION

(45) Date of publication of the patent specification: 12.09.90

(51) Int. Cl.[5]: C07D 493/04, C09K 15/06 // (C07D493/04, 307:00, 307:00)

(21) Application number: 87301409.6

(22) Date of filing: 18.02.87

## (54) Method of producing active antioxidant.

(30) Priority: 31.03.86 JP 75361/86

(43) Date of publication of application: 07.10.87 Bulletin 87/41

(45) Publication of the grant of the patent: 12.09.90 Bulletin 90/37

(84) Designated Contracting States: DE FR GB IT

(56) References cited:
EP-A- 0 207 735
US-A- 4 427 694

PATENT ABSTRACTS OF JAPAN, vol. 9, no. 1 (C-259)[1724], 5th January 1985; & JP-A-59 157 173 (TAKEMOTO YUSHI K.K.) 06-09-1984

(73) Proprietor: Takemoto Yushi Kabushiki Kaisha, 2-5, Minato-machi, Gamagouri-shi Aichi-ken(JP)

(72) Inventor: Namiki, Mitsuo, 166-2 Imonoshido Idaka-cho Meito-ku, Nagoya-shi Aichi-ken(JP)
Inventor: Osawa, Toshihiko, 2-5-5-205 Chiyodabashi Chikusa-ku, Nagoya-shi Aichi-ken(JP)
Inventor: Isobe, Minoru, 2-96 Uedayama Tenpaku-ku, Nagoya-shi Aichi-ken(JP)
Inventor: Fukuda, Yasuko, 3-10-1 Ikegami-cho Chikusa-ku, Nagoya-shi Aichi-ken(JP)

(74) Representative: Clifford, Frederick Alan et al, MARKS & CLERK 57/60 Lincoln's Inn Fields, London WC2A 3LS(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of producing an active antioxidant containing a compound which is derived from sesamolin and has the following structural formula (A) (hereinafter referred to as Compound A):

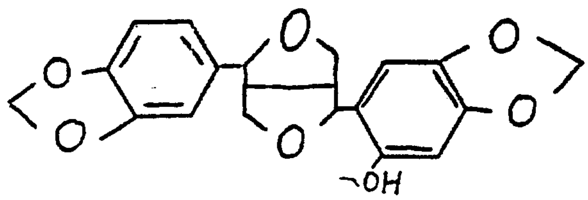

(A)

Sesamol, which is obtained by hydrolysis of sesamolin has long been known as an active antioxidant. The antioxidant activity of sesamol, however, is rather weak. Since sesamol has a low molecular weight, it evaporates at a temperature of about 100°C, and its antioxidant activity is practically non-existent in the range of cooking temperatures from 160 to 180°C at which, for example, frying is done.

Compound A above is a naturally-occurring active antioxidant and its presence in Justicia Simplex, which is used as a medicinal herb in the Himalayan regions, and in sesame seeds, has recently been reported (for example, in Phytochemistry, 19, 322 (1980)).

Methods of obtaining Compound A by extracting and separating it from Justicia Simplex and sesame seeds have been proposed but since the content of Compound A is only about 220ppm in Justicia Simplex and about 15ppm in sesame seeds, it is econimocally not feasible on an industrial scale to rely on such a simple extraction-separation process.

The present inventors have already disclosed that Compound A can be obtained by hydrolysis of a glycoside extracted from sesame seeds with β-glucosidase (Japanese Patent Application Tokkai 59-157173). However. this method also is difficult to apply industrially, because the content of Compound A in the glycoside is small and complicated operations for partial extraction are required.

The present invention sets out to eliminate the aforementioned problems and to provide method of producing an active antioxidant. It further sets out to provide an industrially feasible method of producing the aforementioned Compound A.

The present invention consists in one aspect in a method of producing Compound A by rearrangement of sesamolin in which an acid catalyst is applied to sesamolin with substantially no participation by active hydrogen compound in the reaction, i.e. in the substantial absence of active hydrogen compounds.

The present invention dicloses a method of producing an active antioxidant characterised in that Compound A is obtained by rearrangement of sesamolin (as shown below) by application of an acid catalyst with substantially no participation by active hydrogen compounds in the reaction:

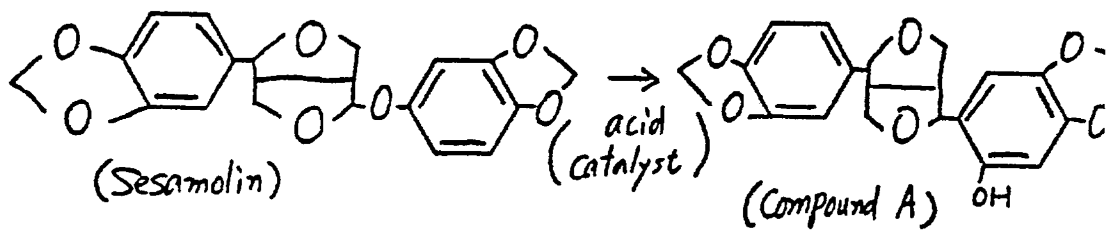


Seasamolin, which is used in the present invention, is present to about 0.15-0.3% by weight in sesame seeds and to about 0.3-0.6% by weight in raw sesame seed oil, obtained by mechanically compressing raw or roasted sesame seeds or by using organic solvent such as hexane for oil extraction from sesame seeds. Sesame seeds and such raw sesame seed oil may therefore be used as a source of sesamolin. Moreover, since a relatively high concentration of sesamolin is also to be present in the distillate (or so-called "deodorized scum") obtained by distillation of raw sesame seed oil with steam under normal or reduced pressure for deodorization, such "deodorized scum" can also be advantageously utilised as a source material for sesamolin.

Sesamolin may be condensed or isolated from such a source material by extraction with a solvent or by a method such as precipitation with cooling, distillation and filtering in a solvent system. Sesamolin of high purity can be obtained by a recrystallization method in a solvent system or by partial extraction type column chromatography (for example, as described in J. of American Oil Chemical Soc., 31, 302 (1954). Thereafter, an acid catalyst is applied to condensed or isolated sesamolin. The acid catalyst for this purpose may be Bronsted acid, a Lewis acid or a solid catalyst having an acid catalyst function. Use may be made, for example, of various inorganic and organic Bronsted acids such as sulphuric acid, phosphoric acid, boric acid, p-toluene sulphonic acid and camphorsulphonic acid, Lewis acids such as aluminium chloride, titanium chloride, tin chloride and boron trifluoride, and solid catalysts with the function of acid catalyst such as acid clay activated clay, zeolite, silica-titanium oxide and cation exchange resins. They may be used singly or two or more of them may be used together as a combination.

There is no particular limitation regarding the amount of acid catalyst to be used, but it is preferably from 0.05 to 10wt% with respect to sesamolin (in the case of a Bronsted acid or a Lewis acid) and from 1 to 50wt% (in the case of a solid catalyst). Excessive amounts of solid catalyst should be avoided because, although the reaction itself is not affected adversely, the Compound A produced in the reaction becomes adsorbed by the catalyst layer and the yield is thereby decreased.

An acid catalyst may be directly applied to condensed or isolated sesamolin at a temperature above its melting point. From the point of view of simplicity of operation, however, it is preferable to dissolve condensed or isolated sesamolin in an inactive solvent such as an aromatic hydrocarbon (e.g. benzene, toluene or xylene) or a halogenated hydrocarbon having no active hydrocarbon group and to then apply an acid catalyst. The acid catalyst is usually applied at a temperature above 50°C, or preferably in the range of 70-200°C. If use is made of an inactive solvent as disclosed above, therefore, the temperature range is from 50°C up to the boiling point of such an inactive solvent. In any case, the acid catalyst need not be dissolved in the reacting system and may be a heterogeneous system. If the reaction is carried out on an industrial scale, however, it is preferable to use a solid acid catalyst because operations such as removal of the catalyst after the reaction can be performed more conveniently.

In order to produce Compound A profitably according to the present invention, it is necessary that the acid catalyst be applied under a condition where no compounds having an active hydrogen group (such as water, alcohols and phenols) participate in the reaction. This is because reactions such as hydrolysis and alcoholysis of sesamolin by such an active hydrogen compound would become the dominant reactions in the presence of an acid catalyst. If water is present, for example, sesamol and samin are produced in the presence of an acid catalyst by hydrolysis of sesamolin. One method of preparing a reaction system wherein active hydrogen compounds do not participate in the reaction is to remove the water and alcohol contents, of the condensed or isolated sesamolin and of the acid catalyst by drying or otherwise removing the solvent before they are used for the reaction. Another method is to add them to an inactive solvent as explained above and then to remove water and alcohols by heating and refluxing for azeotropic distillation.

After such a reaction, Compound A (or a substance containing Compound A) is obtained through processes involving neutralization, filtering, extraction, removal of solvent, etc. The product may be used directly as an active antioxidant or may be subjected to a separation process or the like to improve the concentration of Compound A. Other active antioxidants or synergists may be appropriately mixed together.

The invention will be further described with reference to the following non-limiting Examples.

Example 1

Sesamolin (20g) and toluene (200ml) were placed in a reactor equipped with a stirrer, a thermometer and a condenser with a Dean-Stark separator. After the sesamolin had dissolved, the mixture was heated to remove the water by azeotropic distillation. After the mixture had cooled to 80°C camphorsulphonic acid (0.1g) was added for reaction at 80°C for 60 minutes. The reaction liquid was analyzed by HPLC to study the pea appearing at 11.8 minutes. Compound A was obtained with a yield of 82.4%. The HPLC analysis was carried out with a column of 8mm × 250mm filled with Deverocil ODS-10 (A trade mark, Nomura Chemical Co.Ltd). The solvent was methanol/water=6/4 and its flow rate was 5.0ml/min.

Example 2

Toluene (200ℓ) and cation exchange resin of the sulphonic acid type (2g) were placed in a reactor as described in Example No. 1. The mixture was heated to remove its water by azeotropic distillation. After it had cooled to 80°C, sesamolin (20g) was added. After reaction at 80°C for 30 minutes, the cation exchange resin was removed by filtering and the solvent was removed by means of an evaporator to leave 19g of light brown solid. This was subjected to HPLC analysis as described in Example No. 1 and Compound A was obtained with a yield 73.6%.

Example 3

Sesamolin (20g) and of acid clay (1g) were placed in a reactor and the mixture was heated to 140°C in dry nitrogen vapor. After 30 minutes of reaction with shaking, 18g of light brown solid was obtained by filtering. It was subjected to a similar HPLC analysis and found to contain 12.8g of Compound A.

Example 4

Raw sesame oil was obtained from Chinese sesame seeds by using an expeller. After an aqueous solution of sodium hydroxide was used to remove free fatty acid, the oil was washed with water and then dehydrated. It was further decolored with active charcoal and deodorized at 210°C and 4mm Hg. This so called "deodorized scum" (100g) was dissolved in xylene (500 ml). To this were further added ispopropyl alcohol (300m1) and 1N aqueous sodium hydroxide (200ml). After the mixture was carefully stirred, it was left undisturbed for separation. The isopropyl/water layer was removed and was then washed with water, until the xylene layer became neutral, to remove free fatty acid. A brown semi-solid substance was obtained when a portion of this xylene layer was dried. This was then subjected to a HPLC analysis and found to contain 51% of sesamin and 17% of sesamolin. The aforementioned xylene layer was heated to remove isopropyl alcohol and, after water was further removed by azeotropic distillation acid clay (19g) was added and the mixture was stirred for 30 minutes in reflux. After the mixture was left to cool, the acid clay was removed by filtering and 67g of a brown semi-solid substance was obtained by removing the solvent. This was subjected to a HPLC analysis as in Example No. 1 and found to contain 8.2g of Compound A but no sesamolin.

Example 5

The "deodorized scum" as obtained by the same process of Example No. 4 (100g) was dissolved in benzene (500ml). To this were further added isopropyl alcohol (300ml) and 1N aqueous sodium hydroxide (200ml). After the mixture was carefully stirred, it was left undisturbed for separation. The isopropyl alcohol/water layer was removed, and the remainder was then washed with water until the benzene layer became neutral, so as to remove free fatty acid. Solvent was removed from this benzene layer by an evaporator and 69g of brown solid substance was obtained by drying. After this was dissolved in benzene (200ml) aluminium chloride (0.5g) was added to it for reaction for 30 minutes in reflux. After this had cooled to room temperature, isopropyl alcohol (200ml) and aqueous sodium hydroxide (100ml) were added. The mixture was thoroughly shaken and then left undisturbed to permit separation of an isopropyl alcohol/water layer. The pH of this layer was adjusted to 2 with hydrochloric acid and benzene (200ml) was added After the mixture was thoroughly shaken, it was left quietly and the separated benzene layer was washed until the wahing water became neutral, then hydrated with anhydrous sodium sulfate and then filtered. Solvent was then removed and 8.6 g of light brown solid substance was obtained. This was subjected to a HPLC analysis as in Example No. 1 and found to contain Compound A by 84%.

Comparison Experiment No. 1

Sesamolin (20g) and toluene (200mL) were placed in the same reactor as used in Example No. 1. After they had dissolved the solution was heated to remove water by azeotropic distillation. After the remainder was cooled to 80°C, camphorsulfonic acid (0.1g) and water (5g) were added for a reaction at 80°C for 60 minutes. The reaction liquid was subjected to a HPLC analysis as in Example No. 1 and found to contain 74% sesamol but only a trace of Compound A. A HPLC analysis of sesamol was similarly performed in Example No. 1 except that the solvent was methanol/water=3/7, the flow rate was 4.0ml/min the retention time was 10.4 min.

Comparison Experiment No. 2

This was carried out identically to Comparison Experiment No. 1 except use was made of 5g of ethanol instead of 5g of water. Compound A was obtained in a yield of 13.6%.

Tests on Antioxidation Activity

Compound A isolated by HPLC from the product obtained in Example No. 3 (3.7g, 0.01m mole) the product of Example No. 5 (3.7 ) and, for comparison, sesamolin (0.01m mole), sesamol (0.01 m mole) and dℓ-α-tocopherol (0.01 m mole were taken individually in Erlenmeyer flasks of 100 ml capacity and soya bean oil (20 g) refined by passing through a basic alumina column was added to each flask. After the flasks were thoroughly shaken, they were kept in an oven at 98°C and their peroxide values were measured over periods of time by a known method. The results of measurements are shown in Table 1.

| | Peroxide Value (meq/kg) | | | | | |
|---|---|---|---|---|---|---|
| | 0 | 3 | 5 | 7 | 10 | 15 |
| Compound A | 3.2 | 9.4 | 16 | 26 | 42 | 67 |
| Product in Example 5 | 3.2 | 11 | 17 | 26 | 44 | 78 |
| Sesamolin | 3.2 | 54 | 95 | 137 | 200< | -- |
| Sesamol | 3.2 | 11 | 35 | 62 | 140 | 200< |
| dℓ-α-tocopherol | 3.2 | 13 | 18 | 24 | 40 | 99 |
| none | 3.2 | 59 | 98 | 140 | 200< | -- |

Table 1, therefore clearly shows that superior active antioxidants with Compounds A as a principal ingredient can be produced by a novel, industrially feasible process involving applying an acid catalyst to sesamolin.

## Claims

1. A method of producing an active antioxidant comprising the step of obtaining a compound having the structural formula (A) shown below

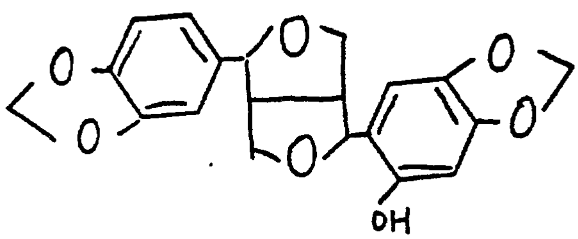

(A)

by applying an acid catalyst to sesamolin in the substantial absence of active hydrogen compounds.

2. The method as claimed in claim 1 wherein said acid catalyst is applied to sesamolin in an inactive solvent without active hydrogen groups.

3. A method as claimed in claim 2 in which the inactive solvent is benzene, toluene, xylene or a halogenated hydrocarbon.

4.The method as claimed in claim 1, 2 or 3 wherein said acid catalyst comprises one or more kinds of solid catalysts with acid catalyst characteristics and selected from a group consisting of acid clay, activated clay, zeolite, silica-titanium oxide and cation exchange resins.

5. A method as claimed in claim 4 in from 1% to 50% by weight of said solid catalyst is used relative to the sesamolin content.

6. The method as claimed in claim 1, 2 or 3 wherein said acid catalyst comprises one or more kinds of organic and/or inorganic Bronsted acid.

7. The method as claimed in claim 1, 2 or 3 wherein said acid catalyst comprises one or more kinds of Lewis acids.

8. A method as claimed inm claim 6 or 7 in which from 0.05 to 10% by weight of said Bronsted acid or Lewis acid is used relative to the sesamolin content.

9. A method as claimed in any one preceding claim in which active hydrogen content is substantially eliminated by drying off or azeotropically distilling off the water and/or alcohol content of the said sesamolin and acid catalyst.

10. A method as claimed in any one preceding claim in which reaction with the acid catalyst is effected at a temperature over 50°C.

11. A method as claimd in claim 10 in which reaction is effected at 70-200°C.

Patentansprüche

1. Verfahren zur Herstellung eines aktiven Antioxidanz, bei dem eine Verbindung mit der unten wiedergegebenen Strukturformel (A) erhalten wird

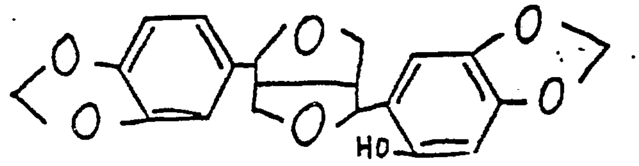

(A)

indem einem sauren Katalysator in einer im wesentlichen vom aktiven Wasserstoffverbindungen freien Umgebung Sesamolin zugefügt wird.

2. Verfahren nach Anspruch 1, bei dem der saure Katalysator dem Sesamolin in einem inaktiven Lösungsmittel ohne aktive Wasserstoffgruppen zugefügt wird.

3. Verfahren nach Anspruch 2, bei dem das inaktive Lösungsmittel Benzol, Toluol, Xylol oder ein halogenierter Kohlenwasserstoff ist.

4. Verfahren nach Anspruch 1, 2 oder 3, bei dem der saure Katalysator mindestens eine Art eines festen Katalysators mit den Eigenschaften eines sauren Katalysators aufweist und ausgewählt ist aus der die Mitglieder saurer Ton, aktivierter Ton, Zeolit, Siliziumtitanoxid und Kationen-Austauschharze enthaltenden Gruppe.

5. Verfahren nach Anspruch 4, bei dem 1 bis 50 Gewichtsprozente des festen Katalysators im Verhältnis zum Anteil an Sesamolin benutzt wird.

6. Verfahren nach Anspruch 1, 2 oder 3, bei dem der saure Katalysator mindestens eine Art einer organischen und/oder inorganischen Brönsted-Säure aufweist.

7. Verfahren nach Anspruch 1, 2 oder 3, bei dem der saure Katalysator mindestens eine Art einer Lewis-Säure aufweist.

8. Verfahren nach Anspruch 6 oder 7, bei dem 0,05 bis 10 Gewichtsprozent der Brönsted-Säure oder Lewis-Säure relativ zum Gehalt am Sesamolin benutzt wird.

9. Verfahren nach einem der vorangegangenen Ansprüche, bei dem der aktive Wasserstoffanteil dadurch im wesentlichen eliminiert wird, daß der Wasser- und/oder Alkoholgehalt des Sesamolin und sauren Katalysators durch Trocknen ode azeotropes Destillieren entfernt wird.

10. Verfahren nach einem der vorangegangenen Ansprüche, dadurch gekennzeichnet, daß die Reaktion mit dem sauren Katalysator bei einer Temperatur oberhalb 50°C ausgeführt wird.

11. Verfahren nach Anspruch 10, bei dem die Reaktion zwischen 70 und 200°C durchgeführt wird.

**Revendications**

1. Procédé de production d'un antioxydant actif comprenant l'étape qui consiste à obtenir un composé répondant la formule développée (A) présentée ci-dessous

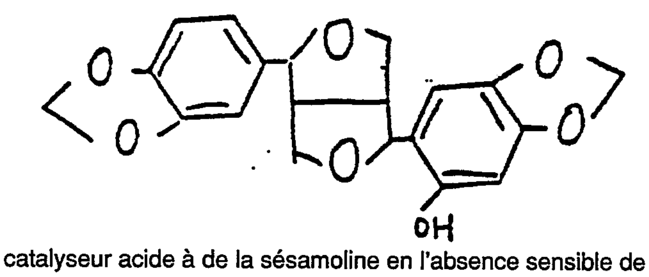

(A)

en appliquant un catalyseur acide à de la sésamoline en l'absence sensible de composés à hydrogène actif.

2. Procédé tel que revendiqué dans la revendication 1, dans lequel ledit catalyseur acide est appliqué à la sésamoline dans un solvant inactif dépourvu de groupes à hydrogène actif.

3. Procédé tel que revendiqué dans la revendication 2, dans lequel le solvant inactif est le benzène, le toluène, le xylène ou un hydrocarbure halogéné.

4. Procédé tel que revendiqué dans la revendication 1, 2 ou 3, dans lequel ledit catalyseur acide comprend un ou plusieurs types de catalyseurs solides ayant des caractéristiques de catalyseur acide et choisis dans le groupe formé par une argile acide, une argile activée, une zéolite, une combinaison siliceoxyde de titane et les résines échangeuses de cations.

5. Procédé tel que revendiqué dans la revendication 4, dans lequel on utilise 1‰ à 50% en poids dudit catalyseur solide par rapport à la quantité présente de sésamoline.

6. Procédé tel que revendiqué dans la revendication 1, 2 ou 3, dans lequel ledit catalyseur acide comprend un ou plusieurs types d'acides de Brönsted organiques et/ou minéraux.

7. Procédé tel que revendiqué dans la revendication 1, 2 ou 3, dans lequel ledit catalyseur acide comprend un ou plusieurs types d'acides de Lewis.

8. Procédé tel que revendiqué dans la revendication 6 ou 7, dans lequel on utilise 0,05 à 10% en poids dudit acide de Brönsted ou dudit acide de Lewis par rapport à la quantité présente de sésamoline.

9. Procédé tel que revendiqué dans l'une quelconque des revendications précédentes, dans lequel la quantité présente d'hydrogène actif est sensiblement éliminée par élimination par séchage ou distillation azéotrope de l'eau et/ou de l'alcool contenus dans ladite sésamoline et ledit catalyseur acide.

10. Procédé tel que revendiqué dans l'une quelconque des revendications précédentes, dans lequel la réaction avec le catalyseur acide est effectuée à une température supérieure à 50°c.

11. Procédé tel que revendiqué dans la revendication 10, dans lequel la réaction est effectuée entre 70°c et 200°c.